(19) Europäisches Patentamt
European Patent Office
Office européen des brevets

(11) Veröffentlichungsnummer : **0 039 861**
**B1**

(12) # EUROPÄISCHE PATENTSCHRIFT

(45) Veröffentlichungstag der Patentschrift : **15.08.84**

(51) Int. Cl.$^3$ : **C 07 J 41/00**, C 07 J 9/00

(21) Anmeldenummer : **81103331.5**

(22) Anmeldetag : **02.05.81**

(54) **Verfahren zum Abbau der 20-Carboxylgruppe in delta-1,4-BNC und verwandten Steroidverbindungen sowie neue stickstoffhaltige C-21-Steroidverbindungen.**

(30) Priorität : **16.05.80 AT 2630/80**
**12.05.80 AT 2536/80**

(43) Veröffentlichungstag der Anmeldung : **18.11.81 Patentblatt 81/46**

(45) Bekanntmachung des Hinweises auf die Patenterteilung : **15.08.84 Patentblatt 84/33**

(84) Benannte Vertragsstaaten : **BE CH FR LI NL**

(56) Entgegenhaltungen :
**US-A- 2 492 188**
**US-A- 4 255 345**
**JOURNAL OF THE AMERICAN CHEMICAL SOCIETY, Band 70, Nr. 3, 3. April 1948, Seiten 887-892 Washington, D.C., U.S.A. P.L. JULIAN et al.: "Sterols. IV. delta20-pregnenes from bisnor-steroid acids"**
**HOUBEN-WEYL METHODEN DER ORGANISCHEN CHEMIE, IV Ed., Band XI/1, Stickstoff Verbindungen II, 1957, Georg Thiem Stuttgart, DE, Seiten 864-866**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Preuss, Wolfgang, Dr.**
**Finkenweg 12**
**D-4019 Monheim (DE)**

**EP 0 039 861 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die offengelegte Europäische Patentanmeldung 79101036.6 (EP-A 004 913) schildert u. a. ein Verfahren zur Herstellung von 17-C-Steroid-α-propionsäureverbindungen — insbesondere zur Herstellung von 3-Oxo-pregna-4-en-20-carbonsäure (Δ4-BNC) und/oder 3-Oxo-pregna-1,4-dien-20-carbonsäure (Δ1,4-BNC) — durch mikrobiellen Seitenkettenabbau an 17-C-Seitenketten-Steroidsubstraten. Durch Verwendung in bestimmter Weise gezüchteter und ausgewählter Mikroorganismen-Defektmutanten, die auch in Abwesenheit von den Steroidringabbau und/oder das Wachstum hemmenden Inhibitoren Steroidverbindungen mit dem 17-C-α-Propionsäurerest liefern, gelingt die Gewinnung von Δ4-BNC und insbesondere Δ1,4-BNC in technischen Mengen. Eine weitere Ausgestaltung dieses Verfahrens ist in der Europäischen Patentanmeldung 79104165.0 (EP-A0 015 308) beschrieben.

In der DE-OS 2 839 033 sind strukturanaloge Steroid-20-carbonsäuren mit einer zusätzlichen Doppelbindung in 9(11)-Stellung sowie Verfahren zu ihrer Herstellung erwähnt. Geschildert ist dort insbesondere eine Möglichkeit zur Herstellung der Δ1,4,9(11)-BNC. Die Δ4,9(11)-BNC ist in der US-PS 4 062 880 beschrieben.

Alle diese BNC-Verbindungen — insbesondere die Δ4-BNC, die Δ1,4-BNC und die Δ1,4,9(11)-BNC — tragen nur in Position 3 eine funktionelle Gruppe im Ringsystem. Alle pharmakologisch wirksamen Corticosteroide enthalten jedoch zusätzliche Sauerstoff-Funktionen. Besonders wichtig sind dabei u. a. die Positionen 11, 17 und 21. Üblicherweise werden einige dieser Sauerstoff-Funktionen chemisch eingeführt, dazu gehören insbesondere die Positionen 17 und 21.

Die Oxydation der Position 11 in Steroidverbindungen erfolgt dagegen bevorzugt mikrobiell. In der Fachliteratur ist eine Vielzahl mikrobieller Steroidoxydationen in position 11 beschrieben. Verwiesen wird in diesem Zusammenhang auf die folgenden Literaturreferate sowie die dort genannten Originalveröffentlichungen :

F. Drawert « Biosynthese von Hydroxy-Verbindungen » ;

Houben Weyl « Methoden der organischen Chemie » (1978) 6/1d, Seiten 378 bis 388 ;

T. H. Stoudt, Adv. Appl. Mikrobiol. 2 (1960) Seiten 190 bis 195 sowie

Handbuch W. Charney und H. L. Herzog « Mikrobial Transformations of Steroids » Academic Press (1967) New York, Seite 29.

Beschrieben wird hier mit zahlreichen Verweisungen auf bestimmte Mikroorganismenstämme, insbesondere aus der Klasse der Fungi, die mikrobielle 11-Hydroxylierung verschiedenartiger Steroidverbindungen und die dabei entstehenden Syntheseprodukte.

Für eine hohe pharmakologische Wirksamkeit ist im allgemeinen die Konfiguration 11β-Hydroxyl oder 11-Oxo erwünscht. Um die in Position 11β-hydroxylierten Steroide zu erhalten, werden entweder Mikroorganismenstämme verwendet, die eine derartige Hydroxylgruppe stereoselektiv einführen, oder es werden andere Mikroorganismen verwendet, die überwiegend oder ausschließlich stereoselektiv in 11α-Position hydroxylieren. In diesem Fall wird zur Gewinnung der 11β-hydroxylierten Steroide zunächst chemisch zum 11-Keton oxydiert und anschließend mit einem geeigneten Reduktionsmittel reduziert, wobei stereoselektiv die 11β-Hydroxyverbindung gebildet werden kann. Zur einschlägigen Literatur dieser anschließenden chemischen Umwandlung wird beispielsweise auf

L.F. Fieser, M. Fieser « Steroide », Verlag Chemie (Weinheim 1961), Seiten 737 ff. sowie die dort zitierte Originalliteraturstelle J. Am. Chem. Soc. 77, 4436 (1955).

Pharmakologisch wichtige Steroidverbindungen bzw. Zwischenprodukte zu ihrer Herstellung — beispielsweise Progesteron, Verbindungen der Cortisonreihe und Verbindungen der Prednisonreihe — enthalten die C-22-Carboxylgruppe nicht mehr, die in den BNC-Verbindungen als Substituent in 20-Stellung vorliegt.

Ältere und parallele Schutzrechte der Anmelderin beschreiben die Umwandlung der 20-Carboxylgruppe in den genannten BNC-Verbindungen zur entsprechenden 20-Säurehalogenidgruppe. Insbesondere die entsprechenden Säurechloride und ihre Herstellung werden geschildert. So beschreibt die ältere Europäische Patentanmeldung 81 100 145.2 (EP-A 34 248) der Anmelderin u. a. das Pregna-1,4-dien-3-on-20-carbonylchlorid (Δ1,4-BNC-Chlorid) und ein Verfahren zu seiner Herstellung. Dieses Säurechlorid — ein funktionelles Derivat der zugehörigen Δ1,4-BNC — eignet sich als Ausgangsmaterial für nachfolgende Reaktionen zur weiteren strukturellen Umwandlung des Seitenkettensubstituenten in 17-Stellung des Steroidringgerüsts. Die Herstellung weiterer Steroid-20-carbonsäurehalogenide der genannten Art ist geschildert in den parallelen Schutzrechtsanmeldungen 81103 332.3 (EP-A 40 347) « Neue Steroid-20-carbonsäureverbindungen und Verfahren zu ihrer Herstellung », Priorität Österreich A 2629/80 vom 16 Mai 1980) sowie 81 103 424.8 (EP-A 39 895) « Neue Pregnan-20-carbonsäurederivate und Verfahren zu ihrer Herstellung », Priorität Österreich A 2535/80 vom 12 Mai 1980).

Die vorliegende Erfindung geht von der Aufgabe aus, ein oder insbesondere mehrfach ungesättigte BNC-20-Carbonsäuren, die auch in der 11-Stellung eine Sauerstoff-Funktion aufweisen können, unter Abbau der Carboxylgruppe in 20-Stellung in C 21-Verbindungen umzuwandeln, die ihrerseits wertvolle Verbindungen der Steroidchemie und insbesondere wertvolle Zwischenprodukte für die Gewinnung von Steroidverbindungen des Progesterontyps und seiner Abkömmlinge sind. Die Erfindung will insbesondere Δ4-BNC, Δ1,4-BNC sowie ihre Derivate mit einer Sauerstoff-Funktion in 11-Stellung und Δ1,4,9(11)-BNC unter Abbau der in 20-Stellung vorliegenden Carboxylgruppe und deren Ersatz durch eine Stickstoff-Funktion in C-21-Steroidverbindungen umwandeln, die ihrerseits wertvolle Zwischenprodukte im Rahmen der Gewinnung pharmakologisch aktiver Steroidderivate sind.

Gegenstand der Erfindung ist dementsprechend in einer ersten Ausführungsform ein Verfahren zur Herstellung von Δ4- und gegebenenfalls wenigstens eine weitere Doppelbindung in 1(2)- und/oder 9(11)-Stellung aufweisenden C-21-Steroidverbindungen mit einer Stickstoff-Funktion in 20-Stellung der allgemeinen Formel I

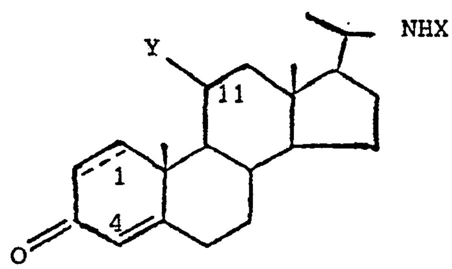

(I)

in der Y Wasserstoff, eine Hydroxylgruppe oder gemeinsam mit dem durch Y substituierten C-Atom eine Carbonylgruppe bedeutet, wobei auch anstelle des Substituenten Y eine 9(11)-en-Bindung vorliegen kann, und in der weiterhin X Wasserstoff, Acyl, einen Kohlensäureesterrest oder gemeinsam mit dem am Stickstoff benachbarten Wasserstoffatom eine Carbonylgruppe bedeutet. Dieses Verfahren der Erfindung ist dadurch gekennzeichnet, daß man das dem gewünschten Reaktionsprodukt strukturanaloge C-20-Carbonsäurehalogenid der allgemeinen Formel II

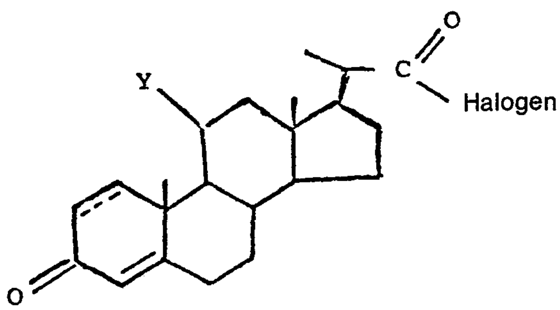

(II)

in der Y die angegebene Bedeutung hat und Halogen bevorzugt Chlor oder Brom bedeutet, in das entsprechende Carbonsäureazid überführt und dieses

a) entweder durch Stickstoffabspaltung zum C-20-Isocyanat umwandelt und gewünschtenfalls dieses in das C-20-Carbamat und/oder das C-20-Amin überführt, oder

b) das Carbonsäureazid unmittelbar in das C-20-Amin umwandelt.

Die einzelnen Stufen und möglichen Abbauschritte des Verfahrens der Erfindung werden schematisch im Formelschema III dargestellt und nachfolgend im einzelnen erläutert. Hierbei sind im Formelschema III der Einfachheit halber lediglich die Seitenkettensubstituenten in 17-Stellung des Steroidringgerüsts dargestellt.

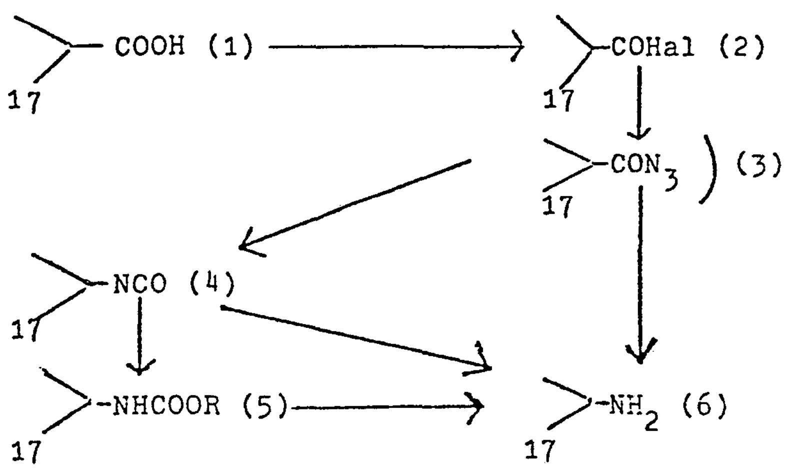

(III)

Im einzelnen gilt zu den Stufen des erfindungsgemäßen Verfahrens das folgende :

Umwandlung der 20-Carbonsäureverbindung (1) zum entsprechenden Carbonsäurehalogenid (2) :

Die Überführung von Carbonsäuren in Säurehalogenide — insbesondere Säurechloride — unter Verwendung von Halogenierungsmitteln wie Phosphorhalogenide, Oxalylhalogenid oder insbesondere Thionylhalogenid ist eine an sich seit langem bekannte und allgemein benutzte Reaktion, auch in der Reihe der 20-Carboxy-Pregnan-Derivate.

Wendet man aber die in der Literatur — siehe hierzu beispielsweise FIAT Final Report No. 996, Seite 24 ff sowie P. L. Julian, E. W. Meyer und H. C. Printy, J. Am. Chem. Soc. *70,* 887 (1948) — für die Umsetzung von 3-Acetoxy-bis-norcholensäure mit Thionylchlorid angegebenen Reaktionsbedingungen auf beispielsweise Δ1,4-BNC an, verestert danach das so erhaltene Säurechlorid mit Methanol und analysiert das Rohprodukt, so findet man im Gaschromatogramm einen zusätzlichen Peak und bei der Elementaranalyse einen deutlichen, zunächst nicht erwarteten Cl-Gehalt.

Ähnliches gilt sogar in noch stärkerem Maße für die Verwendung von Oxalylchlorid anstelle von Thionylchlorid.

Der Grund für das Auftreten dieser unerwünschten Verunreinigungen, die die Ausbeute an gewünschtem Produkt deutlich senken une zu Reinigungsproblemen bei weiteren Umsetzungen mit dem Säurechlorid führen können, ist wahrscheinlich eine Chlorierung mit evtl. anschließender Aromatisierung des A-Ringes im Steroidgerüst, wie sie z. B. für die Umsetzung von Androsta-1,4-dien-3,17-dion (ADD) mit Oxalylchlorid bekannt ist ; siehe hierzu G. W. Moersch et al., J. Org. Chemistry, *29,* 2495 (1964).

Überraschenderweise gelingt die Bildung der erfindungsgemäß gewünschten Säurehalogenide bereits unter außergewöhnlich milden Reaktionsbedingungen, bei denen die unerwünschte Mitreaktion anderer reaktiver Stellen der zugrunde liegenden ein- oder mehrfach ungesättigten BNC-Struktur noch nicht stattfindet. So zeigte sich das überraschende Ergebnis, daß beispielsweise eine praktische quantitative Säurechloridbildung stattfindet, wenn die folgenden Reaktionsbedingungen eingehalten werden : Reaktionstemperaturen unter 15 °C, vorzugsweise unter 5 °C, insbesondere im Bereich von 0-5 °C, stöchiometrische Mengen der Reaktanten oder nur sehr begrenzter Überschuß des Halogenierungsmittels, der vorzugsweise nicht über 20 Molprozent und insbesondere nicht über 10 Molprozent beträgt, sowie Arbeiten in Gegenwart eines inerten Verdünnungsmittels, gewünschtenfalls in Anwesenheit geringer Mengen eines basischen Katalysators.

Als inerte Lösungsmittel kommen beispielsweise halogenierte Kohlenwasserstoffe oder mit Einschränkungen Ether in Betracht. Geeignete inerte Lösungsmittel sind beispielsweise Methylenchlorid oder Chloroform. Als Halogenierungsmittel kommen Phosphorhalogenide, insbesondere $PCl_3$ oder $PCl_5$ bzw. die entsprechenden Bromide, vor allen Dingen jedoch das Thionylhalogenid, insbesondere Thionylchlorid, in Betracht. Katalytische Mengen einer Base, insbesondere Pyridin oder Dimethylformamid, beschleunigen z. B. bei der Δ4-BNC die reaktion, sind aber haufig nicht erforderlich. In Einzelfällen kann die Mitverwendung eines Katalysators wünschenswert sein.

Je nach Wahl der sonstigen Verfahrensparameter bzw. der miteinander umzusetzenden Verbindungen können einzelne Verfahrensparameter auch außerhalb der bis- her angegebenen Werte liegen. So kann beispielsweise die Verfahrenstemperatur im Bereich von etwa − 20 °C bis etwa 75 °C liegen, vorausgesetzt, daß bei den höheren Temperaturen des genannten Bereichs durch geeignete sonstige Verfahrensführung das Entstehen unerwünschter Ringhalogenierungsprodukte vermieden wird. Die Menge des Halogenierungsmittels kann — wiederum geeignete Abstimmung der sonstigen Verfahrensbedingungen vorausgesetzt — auch in nicht unbeträchtlichem Überschuß über die stöchiometrisch benötigte Menge hinaus vorliegen. So sind beispielsweise Mengen bis zu 5 Äquivalenten, vorzugsweise bis zu 3 Äquivalenten des Halogenierungsmittels in Sonderfällen brauchbar. Die Reaktion wird üblicherweise bei Normaldruck durchgeführt. Das Halogenierungsmittel wird zweckmäßigerweise zu der Lösung der umsusetzenden Steroidverbindung im inerten Lösungsmittel gegeben. Es hat sich als vorteilhaft erwiesen, das Halogenierungsmittel in möglichst reiner Form einzusetzen. Offenbar förden üblicherweise im Halogenierungsmittel vorliegende Verunreinigungen unerwünschte Nebenreaktionen. Zweckmäßig ist beispielsweise eine Reinigung des Halogenierungsmittels mit einer ungesättigten Verbindung wie Leinöl oder besonders Squalen. Diese ungesättigten Komponenten reagieren mit den Verunreinigungen im Halogenierungsmittel und senken damit die Bildung unerwünschter Nebenprodukte auf ein Minimum. Bei der Herstellung 11-hydroxylierter Säurehalogenide ist ein Überschuß des Halogenierungsmittels zu vermeiden.

Überführung des Säurehalogenids (2) zum Azid (3) : Grundsätzlich gelten hier die umfangreichen Angaben der Literatur, die insbesondere im Zusammenhang mit dem Curtius-Abbau von Carbonsäuren über deren Azide zu den nächst niederen primären Aminen in der ersten Stufe die Umsetzung von Carbonsäurehalogeniden mit Aziden beschreiben.

Die Carbonsäureazide bilden sich im allgemeinen in glatter Reaktion, siehe hierzu beispielsweise Houben Weyl « Methoden der organischen Chemie » (1957) Bd. XI/1, Seiten 862 ff., insbesondere 864.

Im Rahmen der Erfindung hat sich insbesondere die modifizierte Variante des Curtius-Abbaus unter Anwendung der Zweiphasentechnik bewährt, wie sie — für den Abbau von Fettsäuren und dimerisierten Fettsäuren — in der DE-OS 2 245 611 beschrieben ist. Diese Variante arbeitet mit einer Zweiphasentechnik der Art, daß das Säurehalogenid in einem mit Wasser im wesentlichen nicht mischbaren organischen Lösungsmittel gelöst wird und die Umsetzung des Acylhalogenids und des Metallazids als Zweiphasenreaktion mit einer wäßrigen Lösung des Azids in Gegenwart eines quartären Ammoniumsalzes als Phasentransferkatalysator durchgeführt wird.

Diese Stufe des erfindungsgemäßen Verfahrens wird vorzugsweise bei Bedingungen durchgeführt, die einen wesentlichen Abbau des gebildeten Acylazids zum Isocyanat ausschließen. Vorzugsweise wird bei Temperaturen unter etwa 25 °C und insbesondere bei Temperaturen unter etwa 15 °C zweckmäßig im Bereich von etwa 0-15 °C gearbeitet. Die Reaktionsteilnehmer werden während der Umsetzung vorzugsweise bewegt, z. B. gerührt. Diese Vermischung soll jedoch nicht zur Bildung einer beständigen Emulsion führen. Nach Abschluß der Reaktion kann die das Acylazid enthaltende organische Lösung von der wäßrigen Phase abgetrennt werden. Die organische Phase wird nachgewaschen.

Als mit Wasser nicht mischbare organische Lösungsmittel eignen sich beispielsweise aliphatische, alicyclische oder aromatische Kohlenwasserstoffe oder insbesondere halogenierte Kohlenwasserstoffe, wie Methylenchlorid oder Chlorbenzol. Die Konzentration des Acylhalogenids im Lösungsmittel ist nicht kritisch und kann beispielsweise etwa 5 bis 50 Gewichtsprozent betragen. Als Metallazide werden vorzugsweise Alkalimetall- oder Erdalkalimetallazide, insbesondere Kaliumazid und vor allem Natriumazid eingesetzt. Das Quartäre Ammoniumsalz enthält am Stickstoff vier beliebige organische Reste, beispielsweise Alkyl- oder Arylreste. Vorzugsweise beträgt die gesamte Kohlenstoffzahl nicht mehr als etwa 30, wobei entsprechende Quartärsalze mit bis zu etwa 20 Kohlenstoffatomen in den das Stickstoffatom alkylierenden Resten besonders geeignet sein können. Kurzkettige Alkylreste mit 1-5 Kohlenstoffatomen können besonders bevorzugt sein. Als Anion können beliebige Reste vorliegen. Halogenidionen können besonders zweckmäßig sein. Das quartäre Ammoniumsalz wird üblicherweise in nur geringen Mengen, z. B. Mengen von 0,01-10 Äquivalentprozent, bezogen auf das Säurehalogenid eingesetzt. Die bevorzugten Verfahrenstemperaturen liegen im Bereich unterhalb etwa 10 °C und insbesondere im Bereich von 0-5 °C.

Nach Abschluß der Reaktion, Abtrennung der organischen Phase und ihrem Waschen zur Senkung des Gehalts an quartärer Ammoniumverbindung kann das gebildete Steroid-20-Carbonsäureazid bei vorsichtigem Arbeiten durch Entfernung des Lösungsmittels isoliert werden. Üblicherweise wird jedoch die Lösung des Säureazids nach einem der im folgenden geschilderten Wege unmittelbar weiter verarbeitet.

Umwandlung des Säureazids (3) durch Stickstoffabspaltung zum Isocyanat (4) :

Die thermische Zersetzung der trockenen Lösung des Säureazids im Lösungsmittel — vorzugsweise bei Zersetzungstemperaturen oberhalb von 20 °C — führt unter Stickstoffabspaltung und Molekülumlagerung zum Isocyanat. Hierbei handelt es sich um einen Teilschritt des klassischen Curtius-Abbaus (siehe Houben Weyl aaO., insbesondere Seiten 862 und 865/866). Die thermische Zersetzung wird unter gegebenenfalls ansteigenden Temperaturen durchgeführt, bis die Stickstoffentwicklung im wesentlichen beendet ist. Das gebildete Isocyanat kann isoliert werden, es kann aber auch unmittelbar als Rohprodukt seinen weiteren Umsetzungen zugeführt werden.

Umwandlung der Isocyanate (4) zu den Carbamaten (5) und den Aminen (6) :

Auch hier gelten wieder die allgemein Gesetzmäßigkeiten des Curtius-Abbaues (siehe Houben Weyl aaO., insbesondere Seiten 865/866). Die Umsetzung der Isocyanate mit Alkoholen führt zu den entsprechenden Carbamaten (5). Als Alkohole eignen sich beliebige gesättigte, ungesättigte, aromatische, geradkettige und verzweigte Alkohole, wobei gilt, daß der Kohlenwasserstoffrest des Alkohols vorzugsweise nicht mehr als 20 C-Atome und insbesondere nicht mehr als 10 C-Atome aufweist. Besonders geeignete einfache Vertreter sind niedere Alkanole, beispielsweise die $C_1$ bis $C_4$-Alkohole.

Die Carbamate (5) können durch Verseifung zu den Aminen (6) umgewandelt werden. Für diese Verfahrensstufe eignen sich allerdings weniger die Δ1,4-Verbindungen, da hier die Gefahr besteht, daß unter den Verfahrensbedingungen der stark sauren, beispielsweise stark salzsauren Hydrolyse der A-Ring des Steroidsystems angegriffen wird.

Die Isocyanate (4) lassen sich durch vorzugsweise wäßrig saure Zersetzung unter Decarboxylierung unmittelbar in die Amine (6) umsetzen. Geeignet sind als Reaktionsmedium für diese Verfahrensstufe beispielsweise wäßrige Carbonsäuren, insbesondere wäßrige Essigsäure. Aus dem hierbei gebildeten Aminsalz kann im abschließenden Schritt durch Behandlung mit Basen, beispielsweise Alkalihydroxid, das Amin freigesetzt und isoliert werden. Direkte Umwandlung des Azids (3) zum Amin (6) :

In einer besonders einfachen Reaktion kann das Carbonsäureazid (3) unmittelbar in das C-20-Amin (6) umgewandelt werden. Bekannt ist, daß sich Carbonsäureazide durch Erhitzen mit wäßrigen Säuren, insbesondere wäßriger Essigsäure, unmittelbar zu den Aminen abbauen lassen. Aus den primär anfallenden Aminsalzen werden auch hier die freien Amine durch Umsetzung mit starken Basen, insbesondere Akkalihydroxid, freigesetzt (siehe auch hier Houben Weyl aaO., insbesondere Seiten 870/871). Im einzelnen kann beispielsweise folgendermaßen vorgegangen werden : Die Lösung des gebildeten Säureazids im mit Wasser nicht mischbaren Lösungsmittel wird zu einem Überschuß an wäßriger Essigsäure (Konzentration beispielsweise 50-80 Gewichtsprozent) getropft. Gleichzeichzeitig wird aus der Reaktionsmischung das Lösungsmittel vorsichtig abdestilliert. Es kann unter Umständen zweckmäßig sein, das zusammen mit dem Lösungsmittel entfernte Wasser zu ersetzen. Schließlich steigert man die Reaktionstemperatur auf 60-70 °C. Hat die Gasentwicklung nachgelassen, unterwirft man die Mischung einer Wasserdampfdestillation. Der Rückstand wird im Vakuum eingeengt. Mit Wasser nicht mischbare Lösungsmittel und wäßrige Alkalilauge werden zugesetzt und das Gemisch bewegt.

Anschliessend erfolgt eine Phasentrennung, die organische Phase wird mit Wasser gewaschen und schließlich zur Trockne eingeengt. Das als kristalliner Feststoff anfallende rohe Amin ist im allgemeinen ohne weitere Reinigung zur Umwandlung der Aminogruppe in eine Carbonylgruppe geeignet.

Von den im Rahmen der Erfindung herzustellenden Verbindungen sind unter anderem die Derivate der Δ1,4,9(11)-BNC der allgemeinen Formel I neue Verbindungen und fallen als solche in den Gegenstand der Erfindung. Insbesondere ist damit ein weiterer Gegenstand der Erfindung das Pregna-1,4,9(11)-trien-3-on-20-amin und die entsprechenden Verbindungen mit der Isocyanatgruppe und der Carbamatgruppierung in 20-Stellung.

Auch die erfindungsgemäß herzustellenden Steroidverbindungen mit einer Sauerstoff-Funktion in 11-Stellung sind neu.

Gegenstand der Erfindung sind dementsprechend in einer weiteren Ausführungsform neue C-21-Steroidverbindungen mit einer Sauerstoff-Funktion in 11-Stellung und einer Stickstoff-Funktion in 20-Stellung der allgemeinen Formel IV

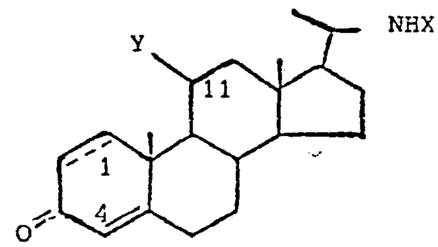

(IV)

in der Y eine Hydroxylgruppe oder gemeinsam mit dem durch Y substituierten C-Atom eine Carbonylgruppe und X Wasserstoff, Acyl, einen Kohlensäureesterrest oder gemeinsam mit dem am Stickstoff benachbarten Wasserstoffatom eine Carbonylgruppe bedeuten.

Die neuen Verbindungen der Erfindung können im A-Ring des Steroidgerüsts einfach ungesättigt sein (Δ4-Verbindungen), sie können aber auch zweifach ungesättigt sein (Δ1,4-Verbindungen). Verbindungen dieses letzten Typs können besonders bedeutungsvoll sein. Die folgende Erfindungsschilderung stellt insbesondere solche Δ1,4-Verbindungen heraus, ist darauch jedoch nicht beschränkt.

Ubereinstimmendes Merkmal dieser neuen Verbindungen ist einerseits ihre Sauerstoff-Funktion in 11-Stellung sowie ihre Stickstoff-Funktion in 20-Stellung.

Die Sauerstoff-Funktion in 11-Stellung kann entweder eine Hydroxylgruppe sein oder es handelt sich um eine Carbonylgruppe, die gemeinsam mit dem durch den Sauerstoff substituierten C-Atom in 11-Stellung gebildet ist. Für die Hydroxylgruppe in 11-Stellung sind die Möglichkeiten der α- und der β-Stellung gegeben. Beide Verbindungstypen fallen in den Rahmen der Erfindung. Die β-Hydroxylverbindungen können aus den eingangs angegebenen Gründen besondere Beteutung haben.

Für die Stickstoff-Funktion in 20-Stellung sind die genannten Möglichkeiten gegeben. Bedeutet X in den Verbindungen der allgemeinen Formel IV Wasserstoff, dann liegt in 20-Stellung die Aminogruppe-$NH_2$ vor. Diese Aminogruppe kann in 20α- oder in 20β-Konfiguration vorliegen. Beide Typen der Stellungsisomeren fallen in den Rahmen der Erfindung. Aus präparativen Gründen kann hier der α-Stellung eine besondere Bedeutung zukommen.

Die 20-Aminoverbindungen lassen sich durch Acylierung leicht in entsprechende Acylamidoverbindungen umwandeln. X aus der Formel IV bedeutet in diesem Fall den Rest RCO—, worin R einen beliebigen Kohlenwasserstoffrest darstellen kann. Auch diese Umsetzungsprodukte sind neue Verbindungen und fallen in den Rahmen der Erfindung.

Eine weitere Gruppe neuer Verbindungen gemäß der Erfindung sind die 20-Carbamatverbindungen, d. h. Verbindungen der allgemeinen Formel IV, in denen X einen Kohlensäureesterrest bedeutet. Sowohl für diese 20-Carbamatoderivate wie für die zuvor genannten Acylamidoverbindungen gelten die Strukturisomerien (20α- bzw. 20β-Stellung), wie sie für die 20-Aminoverbindungen angegeben sind.

Schließlich betrifft die Erfindung 20-Isocyanatoderivate der allgemeinen Formel IV. Hier liegt also in 20-Stellung die Isocyanatgruppe —NCO vor, d. h. X in der allgemeinen Formel bedeutet gemeinsam mit dem benachbarten Wasserstoff am Stickstoff die CO-Gruppierung.

In allen hier geschilderten Fällen kann der A-Ring des Steroidgerüstes einfach (Δ4) oder zweifach (Δ1,4) ungesättigt sein. Für alle Typen gilt weiterhin, daß Y die verschiedenen zuvor angegebenen Bedeutungen aufweisen kann.

Eine besonders bevorzugte Klasse der neuen erfindungsgemäßen Verbindungen sind die 20-Amino-pregna-1,4-dien-3-on-Verbindungen der allgemeinen Formel V

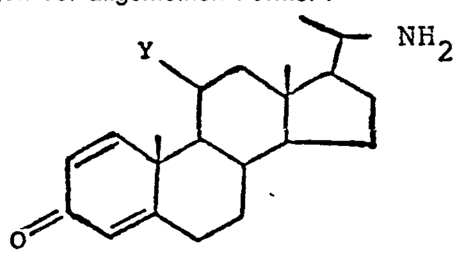

(V)

in der Y eine Hydroxylgruppe und dabei insbesondere β-Hydroxylgruppe oder gemeinsam mit dem durch Y substituierten C-Atom eine Carbonylgruppe bedeutet. Auch die entsprechende Δ4-Verbindung fällt in diese Klasse der besonders bevorzugten Verbindungen gemäß der Erfindung. Amine der hier geschilderten Art sind unter anderem nützliche Zwischenprodukte zur Herstellung von Steroidverbindungen mit der Acetylseitenkette des Progesterons gemäß

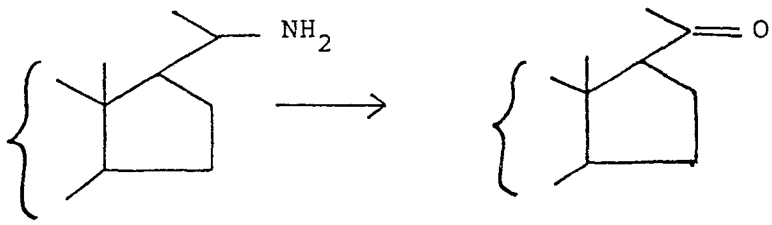

Besonders wichtige Vertreter der hier betroffenen Unterklasse sind beispielsweise das 20-Amino-pregna-1,4-dien-3,11-dion sowie das 20-Amino-11β-hydroxy-pregna-1,4-dien-3-on.

Die erfindungsgemäßen 20-Isocyanato-pregna-1,4-dien-3-on-Verbindungen entsprechen der allgemeinen Formel VI

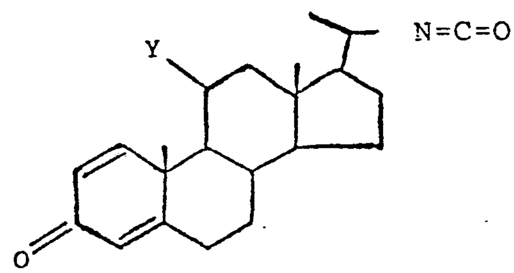

(VI)

in der Y wiederum eine Hydroxylgruppe — vorzugsweise die β-Hydroxylgruppe — oder gemeinsam mit dem durch Y substituierten C-Atom in 11-Stellung eine Carbonylgruppe bedeutet. Entsprechende Δ4-Isocyanatoverbindungen fallen in den Bereich wichtiger erfindungsgemäßer Stoffe. Besonders interessante Beispiele der Stoffklasse sind etwa 20-Isocyanato-pregna-1,4-dien-3,11-dion und 20-Isocyanato-11β-hydroxy-pregna-1,4-dien-3-on.

Die 20-Isocyanato-Verbindungen sind ihrerseits Ausgangspunkt für die entsprechenden Carbamidsäurederivate, die durch Addition von Alkoholen an die Isocyanatgruppe in an sich bekannter Weise leicht gewonnen werden können.

Die entsprechenden 20-Carbamato-Pregna-1,4-dien-3-on-Verbindungen entsprechen der allgemeinen Formel VII

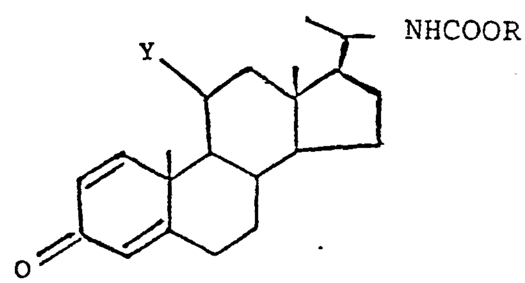

(VII)

Hierbei hat Y wieder die Bedeutung einer Hydroxylgruppe, insbesondere der β-Hydroxylgruppe oder es gilt, daß Y gemeinsam mit dem durch Y substituierten C-Atom in 11-Stellung eine Carbonylgruppe bedeutet. Charakteristische Vertreter dieser Stoffklasse sind 20-Carbamatopregna-1,4-dien-3,11-dion und 20-Carbamato-11β-hydroxypregna-1,4-dien-3-on.

Hat in den Verbindungen der Allgemeinen Formel I der Rest X die Bedeutung RCO—, dann kann R eine beliebige Kohlenwasserstoffgruppe sein. Üblicherweise handelt es sich um einen Alkyl-, Aryl-, Alkaryl- oder Aralkylrest. Vorzugsweise besitzt dieser Rest nicht mehr als 20 C-Atome, insbesondere nicht mehr als 10 C-Atome. Besonders bevorzugt können entsprechend geradkettige oder verzweigte Alkylreste bis zu 5 Kohlenstoffatomen sein, sowie Arylreste mit einem aromatischen Ring, bzw. entsprechende Alkaryl- oder Alkylreste. Der Acetylrest ist zur Identifizierung der Verbindungen ein besonders geeigneter Rest.

Die erfindungsgemäß herzustellenden Steroidverbindungen sind wertvolle Produkte der Steroidchemie. Sie eignen sich insbesondere für weitere chemische Transformationen am Substituenten in 17-Stellung und/oder am Steroidringgerüst. Auf diese Weise können beispielsweise bekannte pharmakologisch aktive Verbindungen bequem zugänglich werden.

Beispiel 1

20-Amino-pregna-1,4-dien-3,11-dion

a) 1 g $\Delta^{1,4}$-11-Oxo-BNC in 20 ml trockenem Methylenchlorid wird bei 0 °C mit 0,25 ml frisch über Squalen destilliertem Thionylchlorid und einem Tropfen Pyridin versetzt und 70 Minuten gerührt.

Danach werden $CH_2Cl_2$ und überschüssiges $SOCl_2$ bei 0 °C im Vakuum entfernt. Der Rückstand wird mit 5 ml abs. $CH_2Cl_2$ versetzt und nochmals zur Trockne eingeengt. Als Rückstand verbleibt 1 g des Carbonsäurechlorids.

b) Das Säurechlorid wird in 20 ml trockenem Methylenchlorid gelöst und unterhalb von 10 °C mit einer Lösung von 273 mg Natriumazid in 3 ml Wasser sowie 10 mg Tetrabutylammoniumchlorid als Phasentransferkatalysator versetzt und 30 Minuten gerührt. Danach wird die wäßrige Phase abgetrennt und man wäscht die $CH_2Cl_2$-Phase noch 2x mit wenig Wasser unterhalb von 10 °C.

c) Die Lösung der Carbonsäureazids in $CH_2Cl_2$ gibt man zu 25 ml 70 %iger Essigsäure und erwärmt langsam, bis $CH_2Cl_2$ abdestilliert. Beim Erwärmen läßt sich eine Gasentwicklung beobachten. Nachdem die Hauptmenge des Methylenchlorids abdestilliert ist, hält man noch 1 h bei ca. 60 °C. Danach wird mit 20 ml Wasser verdünnt und unter Vakuum am Rotationsverdampfer eingeengt.

Der schmierige Rückstand wird in Methylenchlorid aufgenommen, mit einem Überschuß 0,5 n Natronlauge versetzt und über Nacht gerührt. Danach trennt man die Phasen, wäscht die organische Phase mit Wasser nach, trocknet ($Na_2SO_4$) und engt zur Trockne ein. Ausbeute : 850 mg (92 %) rohes Amin.

IR (KBr) : 1 700 (11-Oxo), 1 663 (3-Oxo), 1 618 und 1 600 (1(2) und 4(5) Doppelbindung) $cm^{-1}$.

Beispiel 2

20-Acetamido-pregna-1,4-dien-3,11-dion

500 mg des nach Beispiel 1 hergestellten Amins in 10 ml abs. $CH_2Cl_2$ werden bei Raumtemperatur mit 0,3 ml Acetanhydrid, 0,33 ml Triethylamin und 5-10 mg 4-(N,N-Dimethylamino)-pyridin versetzt. Nach 2 h zeigt ein Dünnschichtchromatogram (Kieselgel ; $CH_2Cl_2$/Essigester/$CH_3OH$ 5 : 4 : 1), daß die Umsetzung abgeschlossen ist. Man wäscht nacheinander mit kalter 2n HCl, $NaHCO_3$-Lösung und Wasser, trocknet über $Na_2SO_4$ und engt zur Trockne ein. Als Rückstand verbleiben 460 mg des Produkts, das dünnschichtchromatographisch nur geringe Verunreinigungen zeigt.

Zur weiteren Reinigung wurde zunächst mit Ether digeriert und danach eine kleine Menge des dabei erhaltenen weißen kristallinen Produkts einer Feinreinigung durch präparative Hochdruckflüssigchromatographie unterzogen.

Fp (nach Umfällen aus Isopropanol/Ether) : 195-202 °C

$C_{23}H_{31}NO_3$

gef. : C 74,35 %, H 8,18 %, N 3,62 %

ber. : C 74,76 %, H 8,46 %, N 3,79 %

Beispiel 3

20 Amino-11 β-hydroxy-pregna-1,4-dien-3-on

Die Reaktion wurde in gleicher Weise durchgeführt, wie in Beispiel 1 für die Darstellung des entsprechenden 11-Oxo-amins beschrieben.

Ausbeute : Aus 1 g $\Delta^{1,4}$-11 β-OH-BNC II 785 mg (85 %) rohes, kristallines Produkt der gesuchten Konstitution.

IR (KBr) : 1 660 (3-Oxo), 1 618 und 1 600 (1(1)- und 3(4)-Doppelbindung) $cm^{-1}$.

Beispiel 4

20-Acetamido-11 β-hydroxy-pregna-1,4-dien-3-on

500 mg des Amins aus Beispiel 3 wurden in ähnlicher Weise wie in Beispiel 2 beschrieben umgesetzt ; zur Vermeidung der Acetylierung der 11β-OH-Gruppe wurde allerdings Pyridin anstelle von Triethylamin/Dimethylaminopyridin verwendet. Die Aufarbeitung und Isolierung erfolgte ebenfalls analog Beispiel 2.

Der nach Abziehen des Lösungsmittels verbliebene Rückstand kristallisierte nach Behandlung mit

Ether. Ausbeute: 465 mg des Produktes, das nach dünnschichtchromatographischer Analyse nur geringfügig verunreinigt war.

Die Feinreinigung wurde wiederum durch präparative Hochdruckflüssigchromatographie durchgeführt.

Fp (umkristallisiert aus Isopropanol): 271-278 °C (Zers.); ab 264 °C bereits teilweise Braunfärbung

$C_{23}H_{33}NO_3$

gef.: C 74,13 %, H 8,70 %; N 3,65 %

ber.: C 74,36 %, H 8,95 %; N 3,77 %

## Beispiel 5

11-Oxo-methylcarbamat

a) Aus 1 g $\Delta^{1,4}$-11-Oxo-BNC wird nach Beispiel 1, abschnitt a) und b), die Lösung des Säureazids in Methylenchlorid hergestellt. Diese Lösung wurde über Molekularsieb 4 A getrocknet und über Nacht stehen gelassen. Danach zeigt das IR-Spektrum, daß die Umlagerung zum Isocyanat abgeschlossen ist (Nachweis durch die Isocyanat-Bande bei 2 260 $cm^{-1}$).

Ir-Daten für 20-Isocyanato-pregna-1,4-dien-3,11-dion (in $CH_2Cl_2$);

2 260, 1 708, 1 662, 1 622, 1 602 $cm^{-1}$

b) Nach Entfernen des Trockenmittels wird ein Teil des $CH_2Cl_2$ abdestilliert, danach gibt man 15 ml abs. Methanol zu und destilliert die restliche $CH_2Cl_2$-Menge ab. Nach insgesamt 4 h Reaktionszeit, zuletzt in siedendem Methanol, zeigt ein Dünnschichtchromatogramm die Beendigung der Reaktion. Nach Einengen zur Trockne erhält man 920 mg gelblich-weißen, kristallinen Feststoff. Das Dünnschichtchromatogramm zeigt nur geringe Mengen an Verunreinigungen. Ca 100 mg dieser Substanz wurden zunächst durch präparative Dünnschichtchromatographie (2 mm Kieselgel, Toluol/Butanon 60 : 40), danach durch Umkristallisieren aus Isopropanol gereinigt.

FP (krist. aus Isopropanol): 188-192 °C

$C_{23}H_{31}NO_4$

gef.: C 71,95 %; H 7,92 %; N 3,52 %

ber.: C 71,66 %; H 8,11 %; N 3,63 %.

## Beispiel 6

11β-OH-Methylcarbamat

a) 20-Isocyanato-11 β-hydroxy-pregna-1,4-dien-3-on: Die Darstellung erfolgte aus $\Delta^{1,4}$-11β-OH-BNC analog Beispiel 5, Abschnitt a).

IR (in $CH_2Cl_2$): 2 262, 1 660, 1 620, 1 601 $cm^{-1}$

b) Die weitere Umsetzung zum 11β-OH-Methylcarbamat erfolgte in gleicher Weise, wie in Beispiel 5, Abschnitt b) für die entsprechende 11-Oxo-Verbindung angegeben.

Aus 1 g $\Delta^{1,4}$-11β-OH-BNC wurden 820 mg rohes Carbamat erhalten.

Fp (nach Umfällen aus Isopropanol/Ether): Die Substanz schmolz bei 148 °C, kristallisierte bei weiterem Aufheizen wieder aus (~150-160 °C) und schmolz schließlich bei 220-224 °C.

$C_{23}H_{33}NO_4$

gef.: C 71,41 %; H 8,35 %; N 3,48 %

ber.: C 71,29 %; H 8,58 %; N 3,61 %.

## Beispiel 7

Herstellung von 20-Amino-pregna-1,4-dien-3-on

a) Pregna-1,4-dien-3-on-20-carbonylchlorid;

17 g (50 mmol) Δ1,4-BNC (1) in 100 ml abs. $CH_2Cl_2$ werden bei 0 °C mit 4,0 ml (55 mmol) frisch über Squalen destilliertem Thionylchlorid versetzt und 20 min bei 0 °C gerührt. Danach werden das Lösungsmittel und überschüssiges Thionylchlorid bei der gleichen Temperaturum Vakuum entfernt. Der Rückstand wird wieder in Methylenchlorid aufgenommen und die Lösung nochmals zur Trockne eingeengt. Der Rückstand ist für weitere Umsetzungen zu verwenden. Um ein zur Analyse geeignetes Säurechlorid zu erhalten, wird das rohe Säurechlorid mit abs. Ether digeriert und nach dem Abziehen des Ethers sorgfältig an der Ölpumpe getrocknet.

| | | gef. | ber. |
|---|---|---|---|
| Elementar-Analyse: | C | 73,0/72,9 | 73,2 |
| | H | 8,01/7,95 | 8,10 |
| | Cl | 9,72/9,51 | 9,82 |

Die Chlorid-Bestimmung nach Hydrolyse einer Probe ergab 9,75 % Chlor als Cl (berechnet 9,82 %).

Fp. (des rohen Säurechlorids) : 142 °C Zersetzung nach Sintern bei 135-138 °C.

b) Aus 25 g Δ1,4-BNC (1) wird gemäß a) das Säurechlorid (2) hergestellt und in 150-200 ml abs. $CH_2Cl_2$ gelöst. Zu dieser Lösung gibt man bei 0 °C 5,8 g Natriumazid in ca. 15 ml Wasser und 150 mg Tetrabutylammoniumchlorid als Phasentransferkatalysator. Es wird 20 min. bei 0 °C nachgerührt. Danach versetzt man mit 20 ml Eiswasser, trennt die Phasen und wächst mit wenig Eiswasser nach.

Die Lösung des Säureazids (3) in $CH_2Cl_2$ wird zu etwa 300-350 ml 70 %iger Essigsäure getropft. Gleichzeitig wird aus der Reaktionsmischung Methylenchlorid vorsichtig abdestilliert. (Es ist unter Umständen zweckmäßig, das zusammen mit dem Methylenchlorid entfernte Wasser zu ersetzen.) Man steigert die Reaktionstemperatur schließlich auf 60-70 °C (1/2-1 h), unterwirft die Mischung dann einer Wasserdampfdestillation sind und engt den Rückstand im Vakuum weitgehend ein.

Danach versetzt man ihn mit ca. 200 ml $CH_2Cl_2$ und 200 ml 10 %iger Natronlauge und rührt über Nacht.

Nach der Phasentrennung wird die organische Phase mit Wasser gewaschen, getrocknet und schließlich zur Trockne eingeengt.

Es verbleiben 21,8 g kristalliner Feststoff mit 90 % 20-Amino-pregna-1,4-dien-3-on (6). Das rohe Amin ist ohne weitere Reinigung zur Überführung ins Dehydroprogesteron zu verwenden.

## Beispiel 8

Abbau von Δ1,4-BNC zum entsprechenden Isocyanat (4) (20-Isocyanato-pregna-1,4-dien-3-on)

Die Lösung des Säureazids (3) wird nach Beispiel 7b aus 25 g Δ1,4-BNC hergestellt. Die Methylenchloridlösung des Säureazids (3) wird über Natriumsulfat getrocknet. Eine langsame $N_2$-Entwicklung zeigt die Umlagerung zum Isocyanat an. Diese Umlagerung kann IR-sepktroskopisch verfolgt werden : Im gleichen Maße wie die Azid-Bande bei 2 120 $cm^{-1}$ schwächer wird, verstärkt sich die Isocyanat-Bande bei 2 270 $cm^{-1}$. Die Umlagerung ist auch bei + 4 °C innerhalb von 24 h beendet, sie wird erwartungsgemäß durch vorsichtiges Erwärmen beschleunigt.

Nach Einengen im Vakuum erhält man 23,4 g festes, schwach gelbes Produkt, das zu über 90 % aus dem Isocyanat besteht (IR($CH_2Cl_2$) : 2 270, 1 662, 1 621, 1 602 $cm^{-1}$).

(Das Isocyanat (4) kann, wie in Beispiel 7b für die Lösung des Säureazids (3) beschrieben, in 20-Amino-pregna-1,4-dien-3-on umgewandelt werden.)

## Beispiel 9

Darstellung des Methylcarbamats (5), R = —$CH_3$, aus 20-Isocyanato-pregna-1,4-dien-3-on.

Aus 17 g Δ1,4-BNC wird, wie in den Beispielen 7a und 7b beschrieben, eine Lösung von 20-Isocyanato-pregna-1,4-dien-3-on hergestellt. Dazu gibt man 80 ml abs. $CH_3OH$, destilliert $CH_2Cl_2$ über eine kurze Kolonne ab und erhitzt dann noch 6-8 h am Rückfluß. Der Fortgang der Reaktion läßt sich anhand der Abnahme der Isocyanatbandebei 2 270 $cm^{-1}$ verfolgen.

Nach Abziehen des Methanols im Vakuum erhält man 16,4 g kristallinen Rückstands, der nach dünnschichtchromatografischer Analyse und den spektroskopischen Daten fast reines Methylcarbamat ist.

[1H-NMR (80 MHz, $COCl_3$, δ-Werte) : 0,78 ppm, s (18-$CH_3$), 1,23 ppm, s (19-$CH_3$) ; 1,15 ppm, d (J = 9 Hz) (21-$CH_3$) ; 3,64 ppm, s (0-$CH_3$) ; 3,64 ppm, m (20-CH) ; olefinische Protonen mit Linien zwischen 6,06 und 7,11 ppm].

## Beispiel 10

Herstellung von 20-Amino-pregna-4-en-3-on aus Δ4-BNC.

10 g Δ4-BNC in 80 ml abs. $CH_2Cl_2$ werden bei 0 °C mit 3,49 ml frisch über Squalen destilliertem $SOCl_2$ und 5 Tropfen Pyridin versetzt. Man rührt 2 h bei 0 °C und entfernt danach das Lösungsmittel, $SO_2$, HCl und überschüssiges Thionylchlorid im Vakuum. Der Rückstand wird in wenig trockenem $CH_2Cl_2$ aufgenommen und nochmals zur Trockne eingeengt. Das feste Säurechlorid wird in 100 ml abs. $CH_2Cl_2$ gelöst und danach dem Curtius-Abbau nach dem in Beispiel 2 beschriebenen Verfahren unterworfen.

Man erhält schließlich 7,6 g kristallines Rohprodukt mit 82 % 20-Amino-pregna-4-en-3-on.

## Beispiel 11

Herstellung von 20-Amino-pregna-1,4,9(11)-trien-3-on

5 g 11β-Hydroxy-pregna-1,4-dien-3-on-20-carbonsäure in 125 ml trockenem $CH_2Cl_2$ werden bei 0 °C mit 2,1 ml trockenem Pyridin und 4,2 ml frisch über Squalen destilliertem Thionylchlorid versetzt.

Man rührt 1 h bei 0 °C nach und entfernt das Lösungsmittel $SO_2$, HCl und überschüssiges Thionylchlorid im Vakuum. Man nimmt den Rückstand in 50 ml $CH_2Cl_2$ auf und engt nochmals zur Trockne ein.

Der verbleibende Rückstand besteht neben Pyridin. HCl vor allem aus Pregna-1,4,9(11)-trien-3-on-20-carbonyl-chlorid.

Dieses Säurechlorid löst man in 125 ml $CH_2Cl_2$, wäscht bei 0° einmal mit wenig kaltem Wasser und führt anschließend den Curtius-Abbau nach dem in Beispiel 2 für Pregna-1,4-dien-3-on-20-carbonylchlorid beschriebenen Verfahren durch. Man erhält 3,81 g rohes 20-Amino-pregna-1,4,9(11)-trien-3-on.

Die Identifizierung und Strukturbestätigung erfolgt am besten nach Acetylierung der $NH_2$-Gruppe (siehe Beispiel 12).

Beispiel 12

20-Acetamido-pregna-1,4,9(11)-trien-3-on

2 g des nach Beispiel 11 hergestellten Amins wurden ohne weitere Reinigung in 50 ml trockenem Methylenchlorid gelöst. Dazu gab man bei Raumtemperatur 2 ml Acetanhydrid, 2,94 ml Triethylamin und eine katalytische Menge 4-Dimethylamino-pyridin. Die Reaktionsmischung wurde nacheinander mit 2n $H_2SO_4$, 5 %iger $KHCO_3$-Lösung und Wasser gewaschen, dann über $Na_2SO_4$ getrocknet und schließlich zur Trockne eingeengt.

Man erhält 2 g des erwarteten Acetamids. Das $^1H$-NMR-Spektrum bestätigt das Vorliegen der postulierten Struktur. Das Rohprodukt ist praktisch rein.

$^1H$-NMR ($CDCL_3$, 80 MHz, δ-Werte) :
$18-CH_3$ : 0,74 s
$19-CH_3$ : 1,40 s
$21-CH_3$ : 1,13 d (J = 6,3 Hz)
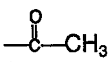 : 1,95
11-CH : 5,50
1-CH, 2-CH, 4-CH : ABC-System mit Linien bei
6,05 ; 6,18 ; 6,20 ; 6,31 ; 6,33 ;
7,13 ; 7,25.

**Ansprüche**

1. Verfahren zur Herstellung von Δ4- und gegebenenfalls wenigstens eine weitere Doppelbindung in 1(2)- und/oder 9(11)-Stellung aufweisenden C-21-Steroidverbindungen mit einer Stickstoff-Funktion in 20-Stellung der allgemeinen Formel I

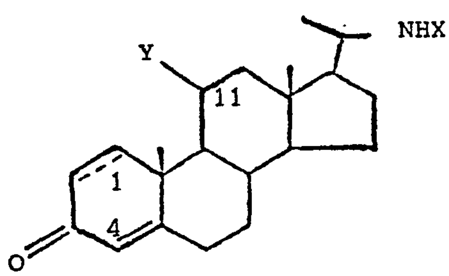

(I)

in der Y Wasserstoff, eine Hydroxylgruppe oder gemeinsam mit dem durch Y substituierten C-Atom eine Carbonylgruppe bedeutet, wobei auch anstelle des Substituenten Y eine 9(11)-en-Bindung vorliegen kann, und in der weiterhin X Wasserstoff, Acyl, einen Kohlenssäureesterrest oder gemeinsam mit dem am Stickstoff benachbarten Wasserstoffatom eine Carbonylgruppe bedeutet, dadurch gekennzeichnet, daß man das das dem gewünschten Reaktionsprodukt strukturanaloge C-20-Carbonsäurehalogenid der allgemeinen Formel II

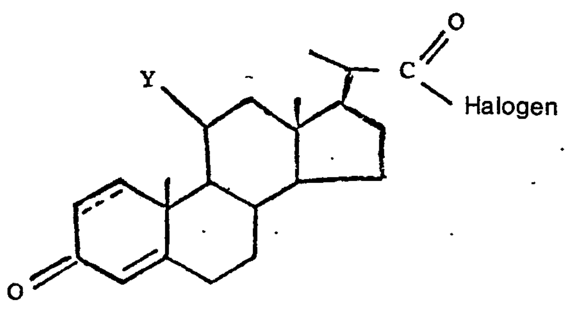

(II)

in der Y die angegebene Bedeutung hat, in das entsprechende Carbonsäureazid überführt und dieses

a) entweder durch Stickstoffabspaltung zum C-20-Isocyanat umwandelt und gewünschtenfalls dieses in das C-20-Carbamat und/oder das C-20-Amin überführt, oder

b) das Carbonsäureazid unmittelbar in das C-20-Amin umwandelt.

2. Verfahren nach Anspruch 1, dadurch gekennzeichnet, daß man die Umsetzung des C-20-Carbonsäurehalogenids zum entsprechenden Azid als wäßrig/organische Zweiphasenreaktion und dabei unter Verwendung von quartären Ammoniumsalzen als Phasentransfer-Katalysatoren durchführt, wobei mit Reaktionstemperaturen unterhalb etwa 25 °C, vorzugsweise im Bereich von 0-5 °C gearbeitet wird.

3. C-21-Steroidverbindungen mit einer Sauerstoff-Funktion in 11-Stellung und einer Stickstoff-Funktion in 20-Stellung der allgemeinen Formel IV

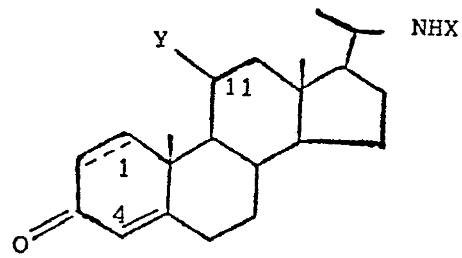

(IV)

in der Y eine Hydroxylgruppe oder gemeinsam mit dem durch Y substituierten C-Atom eine Carbonylgruppe und X Wasserstoff, Acyl, einen Kohlensäureesterrest oder gemeinsam mit dem am N benachbarten Wasserstoffatom eine Carbonylgruppe bedeuten.

4. 20-Amino-pregna-1,4-dien-3-on-Verbindungen der allgemeinen Formel V

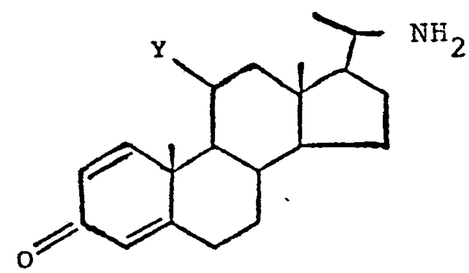

(V)

in der Y eine Hydroxylgruppe, insbesondere die β-Hydroxylgruppe, oder gemeinsam mit dem durch Y substituierten C-Atom eine Carbonylgruppe bedeutet.

5. 20-Amino-pregna-1,4-dien-3,11-dion

6. 20-Amino-11β-hydroxy-pregna-1,4-dien-3-on

7. 20-Isocyanato-pregna-1,4-dien-3-on-Verbindungen der allgemeinen Formel VI

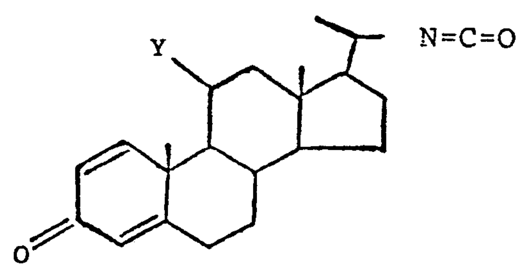

(VI)

in der Y die in Anspruch 4 angegebene Bedeutung hat.

8. 20-Isocyanato-pregna-1,4-dien-3,11-dion

9. 20-Isocyanato-11β-hydroxy-pregna-1,4-dien-3-on

10. 20-Carbamato-pregna-1,4-dien-3-on-Verbindungen der allgemeinen Formel VII

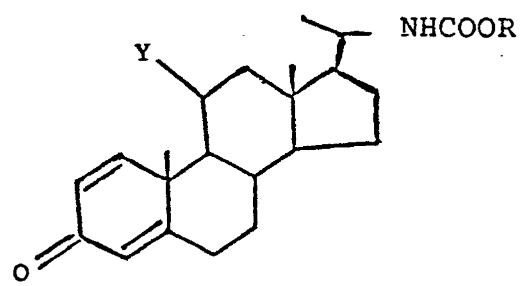

(VII)

in der Y die in Anspruch 4 angegebene Bedeutung hat und R einen Kohlenwasserstoffrest bedeutet.

11. 20-Carbamato-pregna-1,4-dien-3,11-dion
12. 20-Carbamato-11β-hydroxy-pregna-1,4-dien-3-on
13. Pregna-1,4,9(11)-trien-3-on-20-amin bzw. -20-isocyanat.

**Claims**

1. A process for the production of Δ4- C-21-steroid compounds with a nitrogen function in the 20-position and optionally containing at least one other double bond in the 1(2)- and/or 9(11)-position, said steroid compounds corresponding to the following general formula

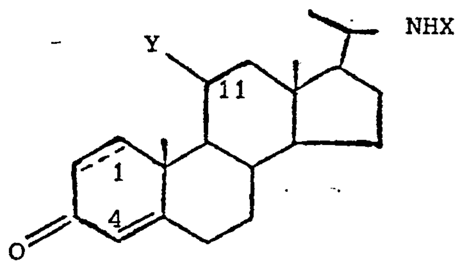

(I)

in which Y represents hydrogen, a hydroxyl group or, together with the C-atom substituted by Y, a carbonyl group, a 9(11)-ene-bond optionally being present instead of the substituent Y, and in which X represents hydrogen, acyl, a carbonic acid ester residue or, together with the adjacent atom on the nitrogen, a carbonyl group, characterized in that the C-20-carboxylic acid halide structurally analogous to the desired reaction product and corresponding to the following general formula

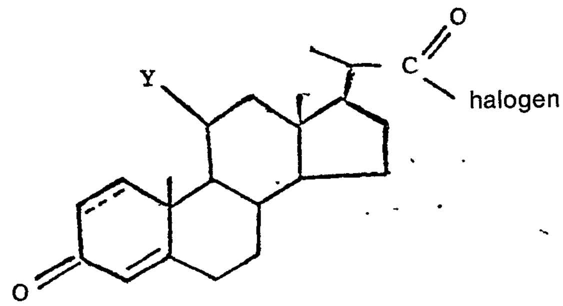

(II)

in which Y is as defined above, is converted into the corresponding carboxylic acid azide which is

a) either converted by the removal of nitrogen into the C-20-isocyanate which, if desired, is converted into the C-20-carbamate and/or the C-20-amine,

b) or is directly converted into the C-20-amine.

2. A process as claimed in Claim 1, characterized in that the reaction of the C-20-carboxylic acid halide to form the corresponding azide is carried out as an aqueous/organic two-phase reaction using quaternary ammonium salts as phase transfer catalysts, the reaction being carried out at temperatures below about 25 °C and preferably at temperatures in the range from 0 to 5 °C.

3. C-21-steroid compounds with an oxygen function in the 11-position and a nitrogen function in the 20-position and corresponding to the following general formula

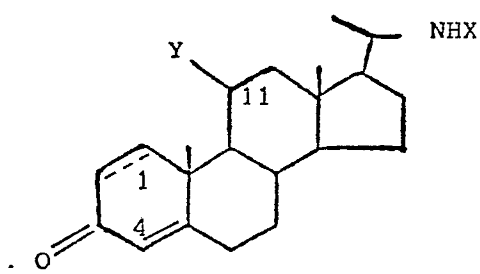

(IV)

in which Y represents a hydroxyl group or, together with the C-atom substituted by Y, a carbonyl group and X represents hydrogen, acyl, a carbonic acid ester residue or, together with the adjacent hydrogen atom on the N, a carbonyl group.

4. 20-amino-pregna-1,4-dien-3-one compounds corresponding to the following general formula

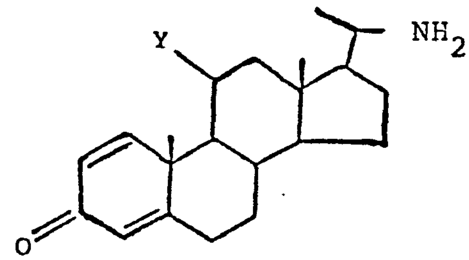

(V)

in which Y represents a hydroxyl group, more particularly the β-hydroxyl group, or, together with the C-atom substituted by Y, represents a carbonyl group.

5. 20-amino-pregna-1,4-dien-3,11-dione.

6. 20-amino-11β-hydroxy-pregna-1,4-dien-3-one.

7. 20-isocyanato-pregna-1,4-dien-3-one compounds corresponding to the following general formula

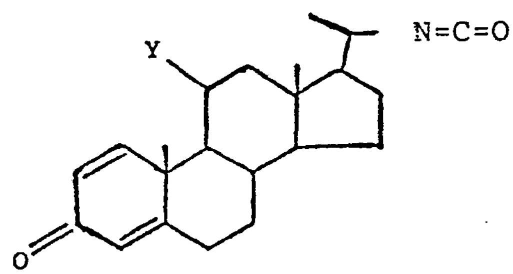

(VI)

in which Y has the meaning defined in Claim 4.

8. 20-isocyanato-pregna-1,4-dien-3,11-dione.

9. 20-isocyanato-11β-hydroxy-pregna-1,4-dien-3-one.

10. 20-carbamato-pregna-1,4-dien-3-one compounds corresponding to the following general formula

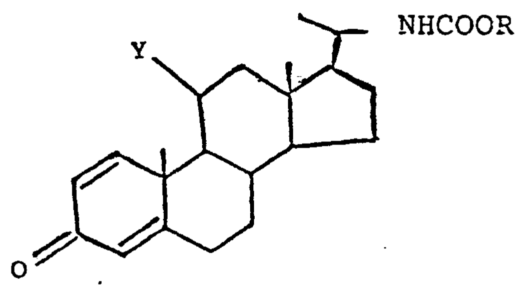

(VII)

in which Y has the meaning defined in Claim 4 and R represents a hydrocarbon radical.

11. 20-carbamato-pregna-1,4-dien-3,11-dione.

12. 20-carbamato-11β-hydroxy-pregna-1,4-dien-3-one.

13. Pregna-1,4,9(11)-trien-3-one-20-amine or -20-isocyanate.

**Revendications**

1. Procédé de préparation de composés stéroïdiques en C 21 présentant une double liaison en Δ4 et le cas échéant au moins une autre liaison en position 1(2) et/ou en position 9(11), avec une fonction azotée en position 20, et répondant à la formule générale I

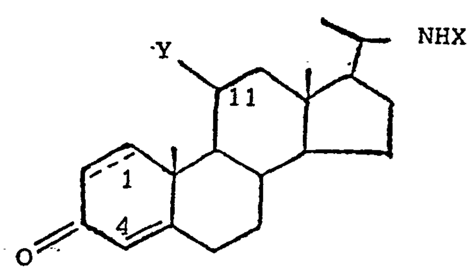

(I)

dans laquelle Y représente l'hydrogène, un groupe hydroxy ou forme un groupe carbonyle avec l'atome de carbone substitué par Y, le substituant Y pouvant également être remplacé par une liaison 9(11)-ène, et X représente l'hydrogène, un groupe acyle, un reste ester d'acide carbonique ou forme en commun avec l'atome d'hydrogène voisin sur l'azote un groupe carbonyle, caractérisé en ce que l'on convertit l'halogénure d'acide carboxylique en C 20 à structure analogue à celle du produit de réaction recherché, répondant à la formule générale II

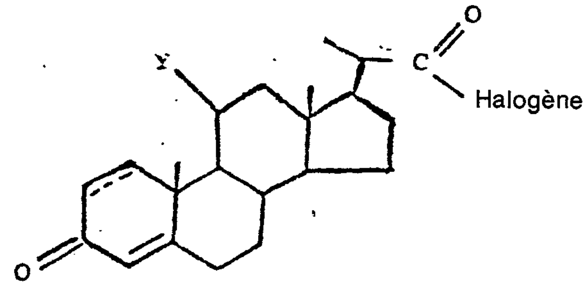

(II)

dans laquelle Y a la signification indiquée ci-dessus, en l'azide d'acide carboxylique correspondant et

a) ou bien on convertit cet acide, par séparation d'azote, en l'isocyanate en C 20 et, si on le désire, on convertit ce dernier en le carbamate en C 20 et/ou l'amine en C 20, ou bien

b) on convertit directement l'azide d'acide carboxylique en l'amine en C 20.

2. Procédé selon la revendication 1, caractérisé en ce que la conversion de l'halogénure d'acide carboxylique en C 20 en l'azide correspondant est effectuée sous la forme d'une réaction à deux phases aqueuse/organique avec utilisation de sels d'ammonium quaternaire en tant que catalyseurs à transfert de phase, avec des températures de réaction inférieures à 25 °C environ et se situant de préférence dans l'intervalle de 0 à 5 °C.

3. Composés stéroïdiques en C 21 ayant une fonction oxygénée en position 11 et une fonction azotée en position 20, de formule générale IV

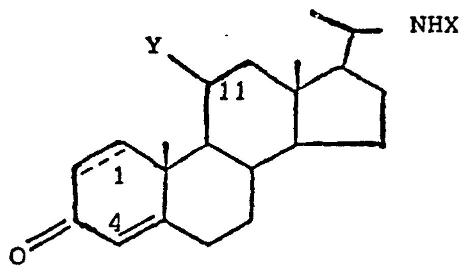

(IV)

dans laquelle Y représente un groupe hydroxy ou forme un groupe carbonyle avec l'atome de carbone substitué par Y et X représente l'hydrogène, un groupe acyle, un reste ester d'acide carbonique, ou forme un groupe carbonyle avec l'atome d'hydrogène voisin sur l'azote.

4. 20-amino-prégna-1,4-diène-3-ones de formule générale V

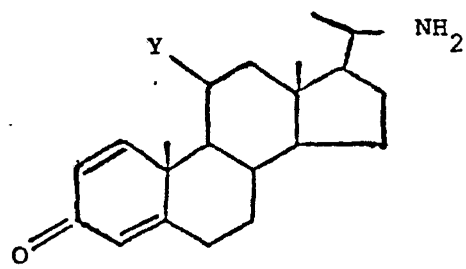

(V)

dans laquelle Y représente un groupe hydroxy, en particulier le groupe β-hydroxy ou forme un groupe carbonyle avec l'atome de carbone substitué par Y.

5. La 20-amino-prégna-1,4-diène-3,11-dione.

6. La 20-amino-11β-hydroxy-prégna-1,4-diène-3-one.

7. 20-isocyanato-prégna-1,4-diène-3-ones de formule générale VI

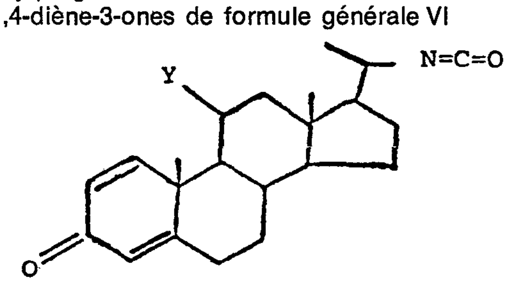

(VI)

dans laquelle Y a la signification indiquée dans la revendication 4.

8. La 20-isocyanato-prégna-1,4-diène-3,11-dione.

9. La 20-isocyanato-11β-hydroxy-prégna-1,4-diène-3-one.

10. 20-carbamato-prégna-1,4-diène-3-ones de formule générale VII

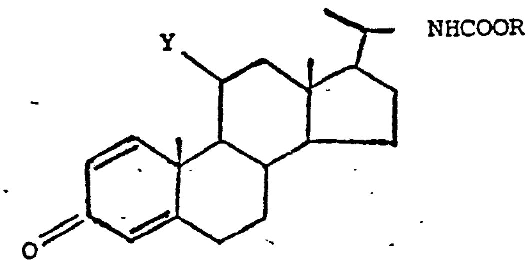

dans laquelle Y a la signification indiquée dans la revendication 4 et R représente un reste hydrocarboné.

11. La 20-carbamato-prégna-1,4-diène-3,11-dione.

12. La 20-carbamato-11β-hydroxy-prégna-1,4-diène-3-one.

13. Le prégna-1,4,9(11)-triène-3-one-20-amine et le prégna-1,4,9(11)-triène-3-one-20-isocyanate.